(19)

Europäisches
Patentamt
European
Patent Office
Office européen
des brevets

(11) **EP 2 199 319 A1**

(12) **EUROPEAN PATENT APPLICATION**

(43) Date of publication:
**23.06.2010 Bulletin 2010/25**

(51) Int Cl.:
**C08J 5/18** *(2006.01)*     **C08L 1/02** *(2006.01)*
**B01D 63/02** *(2006.01)*

(21) Application number: **08022114.6**

(22) Date of filing: **19.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Gambro Lundia AB**
**220 10 Lund (SE)**

(72) Inventors:
• **Krause, Bernd**
**72414 Rangendingen (DE)**

• **Hornung, Markus**
**72147 Nehren (DE)**
• **Göhl, Hermann**
**72406 Bisingen (DE)**

(74) Representative: **Hornung, Veronika Margot et al**
**c/o Gambro Dialysatoren GmbH**
**Holger-Crafoord-Strasse 26**
**72379 Hechingen (DE)**

(54) **Virus filter**

(57)    The invention relates to a virus filter membrane which can be used for the removal of virus particles including parvovirus. The invention further relates to a method for producing the membrane. The membrane comprises cellulose and is prepared from a solution of cellulose in an ionic liquid.

EP 2 199 319 A1

**Description**

**Technical Field**

The invention relates to a virus filter membrane which can be used for the removal of virus particles including parvovirus. The invention further relates to a method for producing the membrane. The membrane comprises cellulose and is prepared from a solution of cellulose in an ionic liquid.

**Background of the Invention**

Virus removal by filtration techniques is known in the art. State of the art virus removal filters in the biotech industry are manufactured from either (i) cellulose (Planova®, Asahi) using the copper solution-based manufacturing process or (ii) synthetic polymers (PES, PSU, PVDF) manufactured by Sartorius, Pall and Millipore. Examples of commercial products used for this purpose are:

- Pall Filtron™ Omega VR filter module comprising a strongly hydrophilic polyethersulfone membrane in a polyurethane cassette format;
- Asahi Planova® filter module comprising a polycarbonate housing and a polyurethane sealant containing an assembly of hollow fibers spun from cuprammonium cellulose solution;
- Pall Ultipor™ VF DV50 filter cartridge comprising three layers of hydrophilic polyvinylidene difluoride membrane on non-woven polyester support in a pleated cartridge with a polypropylene core;
- Millipore Viresolve™ membrane filter module comprising single layers of hydrophilic polyvinylidene difluoride membrane in a TFF open-channel module.

The advantage of the synthetic based filters is the high flux, which on the other side leads to a reduced / varying rejection behavior for virus particles (expressed as LRV). The cellulose-based filter of Asahi shows excellent rejection of parvovirus and high passage of Mab at high feed concentrations, however, the flux that can be achieved with these filters is limited.

There is a need for a membrane combining low price per filter, higher flux than the Asahi products and better rejection behavior than synthetic virus filters.

Various cellulose-based filter systems for virus removal have been described in prior art:

EP 0 798 003 A2 discloses a method of reducing the titer of viruses in a solution of biologically active proteins without adversely affecting the activity of the protein, the method comprising the step of passing the solution at a pH ranging from about 5.0 to about 7.0 through a structured depth filter comprising a positively charged porous filter element comprising a filter aid and a self binding matrix of cellulose fiber, at least one of which are coated with a cationic resin. The average pore diameter of the filter element is within the range of about 0.25 $\mu$m to about 2 $\mu$m.

JP 08/187086 A discloses a porous hollow fiber membrane for virus removal made from cuprammonium regenerated cellulose which has an average pore diameter of 10 to 200 nm and a thickness of 10 to 100 $\mu$m.

J. Membr. Sci. 278 (2006) 3 discloses removal of parvovirus from IgG solutions by a module composed of cuprammonium regenerated cellulose hollow fiber membranes. A virus logarithmic rejection value LRV > 4 was achieved and at an IgG concentration of 5 g/l, hydraulic permeability was about $25 \cdot 10^{-4}$ cm$^3$/(cm$^2 \cdot$bar$\cdot$sec).

EP 0 302 949 A1 discloses a porous hollow fiber membrane for virus removal which is characterized by surfaces having pores with an in-a-plane average pore diameter of 0.01 to 10 $\mu$m and an in-a-plane porosity of not less than 10%. The membrane can be produced from a cuprammonium solution of cellulose.

Recently, ionic liquids have been proposed as alternative solvents for cellulose and processes for preparing membranes comprising regenerated cellulose have been described:

WO 2003/029329 discloses a method for dissolving cellulose in a hydrophilic ionic liquid comprised of cations and anions in the substantial absence of water or a nitrogen-containing base. Particularly preferred cations are 1,3-di-($C_1$-$C_6$ alkyl)-3-methylimidazolium ions. The anions are halides or pseudohalides.

DE 10 2005 062 608 A1 discloses a solution system for carbohydrates, based on molten ionic liquid, optionally containing additives, which contains protic solvent(s) and, if the only protic solvent is water, in an amount more than about 5 wt.%. Preferred cations are 1-ethyl-3-methylimidazolium, 1,3-dimethylimidazolium and 1-butyl-3-methylimidazolium, preferred anions are halides, perchlorate, pseudo-halide, sulfate, phosphate, alkylphosphate and $C_1$-$C_6$ carboxylates.

US 2008/0164440 A1 discloses a solution comprising cellulose, an ionic liquid comprising anions and cations as solvent and 6-30% by weight of a nitrogen-comprising base, based on the total weight of the solution. The ionic liquid comprises a quaternary ammonium cation, an oxonium cation, a sulfonium cation or a phosphonium cation and a monovalent, divalent, trivalent or tetravalent anion.

CN 1491974 A discloses a cellulose solution consisting of ionic liquid and cellulose in the weight ratio of 3-8 to 100. The ionic liquid is 1-ethyl-3-methylimidazol acetate, 1-allyl-3-methylimidazol chlorate, 1-allyl-3-methylimidazol acetate or 1-butyl-3-methylimidazol acetate. The cellulose material is insoluble wood pulp fiber. The cellulose solution is used in producing various regenerated cellulose products.

CN 101089249 A discloses a method for preparing a cellulose polyblend fiber by using ionic liquid as solvent. Said preparation method includes the following steps: (1) uniformly mixing pulverized cellulose, chitin/chitosan and ionic liquid, dissolving them for 1-80 h at 25-160 °C to obtain a uniform stable spinning solution with a total solid content of 3-40%; (2) filtering and de-foaming, then passing the spinning solution through a single-hole or multi-hole spinneret, and into a coagulating bath and solidifying the fiber, at a spinning speed of 5-150 m/min; and (3), plasticizing, drawing, washing, bleaching, oiling and drying the fiber to obtain the cellulose polyblend fiber product. It is proposed that the spinning solution can also be used for preparing hollow fiber membranes for use in artificial organs and in membranes for water purification.

CN 101089250 A discloses a method for preparing bamboo pulp fiber by using ionic liquid as solvent, including the following steps: (1) uniformly mixing the pulverized bamboo pulp and ionic liquid, dissolving for 1-80 h at 35-160 °C to obtain a uniform stable spinning solution with a total solid content of 3-40%; (2) filtering and de-foaming, then passing the spinning solution through a multi-hole spinneret and into a coagulating bath and solidifying the fiber, at a spinning speed of 5-150 m/min; and (3) plasticizing, drawing, washing, bleaching, oiling and drying the fiber to obtain the bamboo pulp fiber product. It is proposed that the spinning solution can also be used for preparing hollow fiber membranes for use in artificial organs and in membranes for water purification.

## Summary of the Invention

The present invention provides a membrane made of cellulose, which can be used for virus removal from liquids. In one embodiment of the invention, the membrane takes the form of a hollow fiber. The membrane is prepared from a solution of cellulose in an ionic liquid, e.g. 1-ethyl-3-methylimidazolium acetate (EMIM OAc). The membrane shows (i) high rejection for gold nano-particles (having 20 nm diameter and simulating parvovirus), (ii) high passage of Mab feed solution (sieving coefficient for IgG $\approx$ 1), (iii) high feed flux (which results in a high feed capacity for this type of membrane), and (iv) low protein adsorption and low fouling, which ensures a stable high filtration rate over time.

## Detailed Description of the Invention

In one aspect of the present invention, a membrane comprising cellulose, having a hydraulic permeability of from $40 \cdot 10^{-4}$ to $500 \cdot 10^{-4}$ cm$^3$/(cm$^2 \cdot$bar$\cdot$sec), preferably of from $50 \cdot 10^{-4}$ to $250 \cdot 10^{-4}$ cm$^3$/(cm$^2 \cdot$bar$\cdot$sec), a sieving coefficient for IgG of greater than 0.8 and a GPR of greater than 1.4, preferably greater than 1.7, more preferably greater than 2.0, most preferably grater than 2.3 is provided. The membrane is copper-free. The term "copper-free" means that the content of copper in the membrane is below the detection limit of XRF (x-ray fluorescence spectroscopy). Preferably, the copper content of the membrane is even below the detection limit of AAS (atomic absorption spectroscopy) or ICP-MS (inductively coupled plasma mass spectrometry), respectively.

In comparison, the commercially available Asahi Planova$^®$ membrane, which is made from regenerated cellulose using the cuprammonium process, shows a hydraulic permeability of about $25 \cdot 10^{-4}$ cm$^3$/(cm$^2 \cdot$bar$\cdot$sec) and, due to its production process, contains residual copper.

The membrane according to the invention can have any suitable geometry according to the needs of the intended use, i.e. it can be used as a flat sheet, a hollow fiber or a bundle of hollow fibers, or can be shaped to form chambers or other geometries needed.

The membrane used in the invention is prepared from cellulose. Examples of suitable types of cellulose for preparing the membranes of the invention are beech sulfite pulp or cotton linters.

The solvents for the cellulose are ionic liquids. Suitable ionic liquids are disclosed in WO 2003/029329 and DE 10 2005 062 608 A1, both incorporated herein by reference. In one embodiment of the invention, the ionic liquids comprise cations selected from 1-ethyl-3-methylimidazolium, 1,3-dimethylimidazolium and 1-butyl-3-methylimidazolium, and anions selected from halides, perchlorate, pseudohalides, sulfate, phosphate, alkyl phosphates and C$_1$-C$_6$ carboxylates. In another embodiment of the invention, the ionic liquids are selected from the group consisting of 1-ethyl-3-methylimidazol acetate, 1-allyl-3-methylimidazol chlorate, 1-allyl-3-methylimidazol acetate, and 1-butyl-3-methylimidazol acetate. In another embodiment, the ionic liquid comprises cations selected from methyl-tri-n-butylammonium, methylimidazolium, 1-ethyl-3-methylimidazolium, 1-butyl-3-methylimidazolium, 1-ethyl-2,3-dimethylimidazolium, 1,2,3-trimethylimidazolium, 1,2,4-trimethylpyrazolium, in particular 1-ethyl-3-methylimidazolium and 1-butyl-3-methylimidazolium; and anions selected from acetate, ethylsulfate, methylsulfate, thiocyanate, hydrogensulfate, tetrachloroaluminate, and chloride, in particular acetate. In a particular embodiment, the solvent is 1-ethyl-3-methylimidazolium acetate (EMIM OAc).

The concentration of cellulose in the solution generally is in the range of from 3 to 25 wt.-%, for instance 4 to 12 wt.-%. In one embodiment, the concentration of cellulose in the solution is 5 to 11 wt.-%. 5 wt.-% solutions of cellulose in 1-ethyl-3-methylimidazolium acetate are commercially available from BASF Aktiengesellschaft under the trade name Cellionics™.

In one embodiment of the invention, the viscosity of the cellulose solution is in the range of from 100,000 mPa·s to 1,000,000 mPa·s, e.g. from 400,000 mPa·s to 700,000 mPa·s for hollow fiber production, and from 150,000 mPa·s to 2,000,000 mPa·s, e.g. from 200,000 mPa·s to 800,000 mPa·s or flat sheet membrane production.

An illustrative manufacturing process of the membranes of the invention comprises the steps of:

(a) extruding a solution of cellulose in an ionic liquid in order to form a hollow fiber or a flat membrane;

(b) simultaneously, in the case of the preparation of a hollow fiber membrane, or after extrusion in the case of the formation of a flat membrane, solidifying the membrane obtained by a phase inversion process by partial or total contact of the extruded product with a liquid or gaseous fluid which is chemically inert with respect to cellulose;

(c) washing the flat membrane or the hollow fiber membrane obtained;

(d) treating the flat membrane or the hollow fiber membrane with a pore stabilizer (e.g. glycerol or a mixture of water and glycerol).

The solution of cellulose in an ionic liquid can be prepared by dissolving cellulose in the solvent and degassing the solution in a drying oven creating a vacuum (approximately 100 mbar).

The temperature of the cellulose solution may vary over a relatively wide range. It is advantageous to choose a temperature ranging from ambient temperature to up to 90 °C, e.g. 60 to 80 °C.

The cellulose solution is then cast as a uniform film onto a smooth surface such as a glass slide which acts as a supporting area by utilizing a special coating knife. The velocity of casting the cellulose film can vary over a relatively wide range. A velocity between 10 and 20 mm/s may be used.

The coating knife with a defined gap height, e.g. 70 to 90 $\mu$m, is driven with constant velocity creating a uniform polymer film. For a good thickness distribution, a coating knife having a uniform gap is advisable. In an exemplary lab-scale process, the cellulose solution is applied steady-going onto a glass slide using a syringe. It is important to work bubble-free.

The precipitation bath preferably consists of $H_2O$ and optionally a solvent. The bath preferably comprises $H_2O$ in amount of from 30 wt.-% to 100 wt.-% and a solvent selected from the ionic liquids mentioned above, e.g. 1-ethyl-3-methylimidazolium acetate, and mixtures thereof in an amount of from 70 wt.-% to 0 wt.-%. In one embodiment of the process, the precipitation bath comprises $H_2O$ in an amount of from 56 to 66 wt.-%, and 1-ethyl-3-methylimidazolium acetate in an amount of from 34 to 44 wt.-%. The addition of solvent to the precipitation bath yields membranes having higher permeability.

The temperature of the precipitation bath generally can be in the range of from 0 to 50 °C. In many cases, ambient temperature will yield satisfactory results. A higher temperature of the precipitation bath yields membranes having higher permeability.

Precipitation time can also be varied over a wide range. Normally, 5 to 15 minutes will be sufficient.

The precipitated membrane is washed, treated with a pore stabilizer, preferably glycerol or a mixture of water and glycerol, in order to stabilize the pores, and can then be stored in a non-solvent until the membranes are cut. After cutting, the membranes can be dried and sterilized.

The cellulose solution can be spun in the form of hollow fibers by various processes which are known to the person skilled in the art. The spinneret is generally of annular shape. It is preferably arranged along a vertical axis. It also possesses an axial orifice allowing air or inert gas, which is advantageously filtered, to be introduced through the spinneret, thus contributing to the support and uniform shaping of the axial channel of the hollow fiber during formation. Instead of air or inert gas, a mixture of a solvent and a non-solvent for cellulose can be introduced through the axial orifice.

The air or the gas can be introduced at ambient temperature and at a pressure of the order of atmospheric pressure or, advantageously, at a slight overpressure, for example at a relative pressure of less than 500 mm of water.

In an exemplary process for the preparation of hollow fibers, a "collodion", formed from a solution of the cellulose in an ionic liquid, is injected into a spinneret with an annular orifice, and then, immediately at the outlet of the spinneret, the inside and the outside of the nascent hollow fiber are coagulated by means of a coagulating fluid which is either water or a mixture of water and an ionic liquid, usually between 1 and 35% by weight.

By "collodion" is meant a spinnable solution of cellulose in an ionic liquid. Instead of a single solvent, the collodion can be produced from a mixture of solvents; it is also possible to add a minor amount of a non-solvent for cellulose to this solvent or mixture of solvents, insofar as the whole remains a solvent for the copolymer.

The concentration of cellulose in the collodion is generally at least 3% by weight and less than that required for saturation; it is preferably less than 20% by weight. In one embodiment of the invention, the concentration of cellulose in the collodion is 5 to 11% by weight, based on the total weight of the collodion.

The collodion is then coagulated by simply bringing the collodion into contact with the coagulating fluid, immediately at the outlet of the spinneret. The temperature difference between the collodion and the coagulating fluid generally is less than 50 °C.

The external coagulation of the nascent hollow fiber is effected in practice by conducting the fiber, during its formation, through a bath of coagulating fluid ("coagulating bath").

The internal coagulation of the nascent hollow fiber is effected in practice by injecting coagulating fluid (e.g. a mixture of a solvent and a non-solvent) into the core, that is to say into the inside of the fiber during its formation. Alternatively, the internal coagulation can be effected by injecting inert gas, e.g. nitrogen, into the core. The injection of inert gas yields membranes having a higher permeability.

The temperature of the coagulating fluids and of the collodion can vary within wide limits; they are generally between 0 and 50 °C, and preferably between 0 and 30 °C. Low temperatures generally favor the absence of vacuoles and also yield membranes showing lower permeability. The temperatures of the collodion, of the internal coagulating fluid and of the spinneret are usually the same for economic and technical reasons; in contrast, the temperature of the coagulating bath can be different from the other three.

During the coagulation, a part of the solvent for the collodion migrates into the coagulating fluid and this, consequently, can change the composition thereof somewhat.

For the purpose of ensuring that the hollow fibers have a uniform and symmetrical shape, it is preferred to position the spinneret along a vertical axis with the collodion flowing in a downwards direction; in practice, the spinnerets are immersed spinnerets.

The nascent fiber is kept in contact with the coagulating fluids at least until the fiber is sufficiently hardened to enable it to be handled and no longer flows under the working conditions.

The coagulation described above can be followed by washing with pure water in order to remove non-polymeric constituents from the fiber (especially solvents and/or salts).

The fibers, prepared by the coagulation process described above, can be given an aqueous heat treatment for the purposes of improving their performance, especially their permeability.

The hollow fibers subjected to this treatment can have undergone a partial washing with pure water or with a mixture of water and an organic solvent but, regardless of this, at the time of the aqueous heat treatment, the fibers still advantageously contain a little of the solvent or solvents which initially formed the collodion; more precisely, the proportion of residual solvent in the hollow fibers during the aqueous heat treatment is generally between 5 and 20%, and preferably between 10 and 17% by weight.

This aqueous heat treatment generally consists of immersing the hollow fibers in water or in a mixture of water and a non-solvent at a temperature between 60 °C and 250 °C, and preferably between 80 °C and 190 °C.

The water or the aqueous mixtures used can be in the vapor phase; however, it is preferable to use them in the liquid phase. Of course, a treatment above 100 °C can make it necessary to work under pressure when it is desired to use liquid water in order to carry out the aqueous heat treatment.

The proportion of water in the aqueous mixtures which can be used in this treatment is usually greater than 50% by weight, and preferably greater than 90%. The water can be mixed with organic solvents or with inorganic or organic electrolytes but it is then preferred to use mixtures which are chemically neutral and especially not strongly basic so as to avoid chemical attack of the cellulose. A pH of 6 to 8 is generally suitable.

According to an advantageous procedure, the aqueous heat treatment is carried out continuously by making the fiber flow continuously through the hot water treatment bath, the pressure being atmospheric and the temperature being at most equal to 100 °C. The duration of the treatment is usually 5 seconds to 5 minutes but there is no critical upper limit.

To provide the fibers with a good dimensional stability on storage, it is advantageous to subject them to a relaxation treatment. To do this, they are passed through one or more baths of water, the temperature of which is above the use and/or storage temperature of the fiber. Generally, the temperature of the bath or baths is from 40 °C to 100 °C and preferably from 80 °C to 100 °C. The hollow fiber is generally guided between the inlet and outlet of the bath by rollers, the relative speeds of which are adjusted so that the fiber is constantly kept immersed and slack.

This relaxation bath also makes it possible to carry out an additional washing of the hollow fiber and to remove the traces of non-polymeric residues. The residence time of the hollow fiber in the relaxation and/or washing bath is generally less than one minute.

There are two ways for producing membranes according to the invention which may be referred to a "wet" and "dry". In case "wet" fibers are prepared, the membranes have to be dried separately in a tube or oven after they have been prepared. To this end, bundles of fibers (for example between 30 and 200 fibers) are placed in a plastic or metal tube. Hot air is passed through this tube within the bundles to dry the fibers.

The hollow fibers can be stored in an aseptic medium, for example in aqueous formaldehyde solution. They can also be impregnated with a water-retention agent, such as ethylene glycol or, preferably, glycerol, for example by immersion into a mixture of water and glycerol containing at least 40% by weight of glycerol, by techniques which are known in the art.

The selectively permeable hollow fibers are of the symmetrical type and have a homogeneous gel-like structure, which is substantially uniform throughout their thickness. The membrane does not allow the passage of virus particles having a diameter of 20 nm or more while it is permeable to antibodies like IgG. The hollow fibers are generally free of vacuoles (empty spaces included in the wall and having a largest dimension of more than about 5 microns). They do not possess a skin or a dense layer on the surface, either on the inside or on the outside.

In a specific embodiment of the present invention, the membrane is a hollow fiber membrane. A hollow fiber membrane of the invention is further characterized by having an inner diameter of from 50 to 2,000 $\mu$m, preferably of from 50 to 1,000 $\mu$m, and more preferably from 100 to 500 $\mu$m. The wall thickness is generally from 3% to 30% of the external diameter, i.e. generally from 5 to 200 $\mu$m, preferably from 10 to 80 $\mu$m, and more preferably from 20 to 70 $\mu$m.

In another specific embodiment of the present invention, the membrane is a flat sheet membrane. The thickness of a flat sheet membrane according to the invention may vary between 10 $\mu$m and 200 $\mu$m. A thickness of 20 $\mu$m to 100 $\mu$m may be especially advantageous for most applications. Usually, several flat sheet membranes will be combined in the form of a stack to form a module for use in the present invention.

The hydraulic permeability of the membranes of the invention, measured at 37 °C, may vary from $40 \cdot 10^{-4}$ to $500 \cdot 10^{-4}$ cm$^3$/(cm$^2 \cdot$bar$\cdot$sec), for instance from $50 \cdot 10^{-4}$ to $250 \cdot 10^{-4}$ cm$^3$/(cm$^2 \cdot$bar$\cdot$sec).

The membranes of the invention have a sieving coefficient for IgG in aqueous solution of at least 0.8, e.g. at least 0.9, and in particular 1.0. The membranes of the invention also show a sieving coefficient of 1.0 for albumin.

The membranes of the invention show a retention of gold nano-particles having 20 nm diameter (simulating porcine parvovirus), GPR (gold particle retention, defined as log(A/F), A/F being the ratio of the gold particle concentration in the feed and in the filtrate), of > 1.4, preferably > 1.7, more preferably > 2.0, in particular > 2.3. The GPR is proportional to the rate of retention of virus particles by the membrane. A GPR of 1.4 corresponds to a reduction of the number of porcine parvovirus in aqueous solution by 3 orders of magnitude, while a GPR of 2.3 corresponds to a reduction by 6 orders of magnitude. The membrane of the present invention can thus be advantageously used for the removal of virus particles from liquids.

Thus, a further aspect of the present invention is a device for removing virus particles from liquids, comprising a membrane of the invention.

The membrane used in the device can take the form of a sheet or a plurality of sheets, e.g. a stack of planar membranes. Alternatively, the membrane used in the device can take the form of a hollow fiber or a plurality of hollow fibers, e.g. a bundle of hollow fiber membranes. Suitable sheets and fibers, respectively, their properties and preparation have been described above.

In one embodiment of the device, the membrane forms an interface between two fluid compartments of the device.

An exemplary device comprises two compartments separated by a semipermeable membrane mounted in a casing, a first internal compartment fitted with two accesses and a second external compartment comprising one or two accesses, both compartments being also separated by a potting compound, based on an appropriate adhesive compound, intended for forming as applicable (i) a cylindrical partition separating both compartments of said device containing a semipermeable membrane of the hollow fiber bundle type as defined above or (ii) a tight seal in said device including a semipermeable membrane of the sheet membrane type as defined above.

Another exemplary device comprises a plurality of hollow fiber membranes, contained within an outer shell, and configured so that fluid within a space external to the hollow fibers (i.e., an extracapillary compartment) is segregated from fluid passing through the hollow fibers and their corresponding orifices. Additionally, the device includes two manifold end chambers within the outer shell on opposite ends of the device. Each of the two lumina of a hollow fiber connects to a different end chamber. The end chambers and the extracapillary compartment are separated by the semipermeable membranes of the hollow fibers. The composition within the extracapillary compartment can be controlled, to a certain extent, by the molecular weight cutoff, or pore size, of the membranes of the hollow fibers.

A further aspect of the invention is a method of removing virus particles from a liquid, comprising filtration of the liquid through a membrane of the invention. In one embodiment of the invention, the filtration is normal-flow filtration (NFF), which is also called dead-ended or direct flow filtration. In another embodiment of the invention, the filtration is tangential-flow filtration (TFF).

The membrane, the method, and the device of the invention can advantageously be used many processes in the biopharmaceutical industry which require removal of virus particles from a liquid.

**Examples**

**Methods**

Preparation of mini-modules

Mini-modules [= fiber bundles in a housing] are prepared by immersion of the fibers in a mixture of glycerol and water (50/50 vol.-%), cutting the bundle to a length of 20 cm, drying the fibers for 1 h at 40 °C and < 100 mbar and transferring the dried fibers into the housing. The mini-module is dried in a vacuum drying oven at 60 °C over night, and then the ends of the fiber bundle are potted with polyurethane. The mini-modules ensure protection of the fibers.
The number of fibers required is calculated for an effective surface A of 10 cm$^2$ according to equation (1)

$$A = \pi \times d_i \times l \times n \; [cm^2] \quad\quad\quad (1)$$

with

$d_i$ = inner diameter of fiber [cm]
n = amount of fibers
l = effective fiber length [cm]

Hydraulic Permeability (Lp) of mini-modules

The hydraulic permeability of a membrane bundle is determined by pressing an exact defined volume of water under pressure through the membrane bundle, which has been sealed on one side, and measuring the required time. The hydraulic permeability can be calculated from the determined time, the effective membrane surface area, the applied pressure and the volume of water pressed through the membrane. From the number of fibers, the fiber length as well as the inner diameter of the fiber, the effective membrane surface area is calculated. The membrane bundle has to be wetted thirty minutes before the Lp-test is performed. For this purpose, the membrane bundle is put in a box containing 500 ml of ultrapure water. After 30 minutes, the membrane bundle is transferred into the testing system. The testing system consists of a water bath that is maintained at 37 °C and a device where the membrane bundle can be implemented mechanically. The filling height of the water bath has to ensure that the membrane bundle is located underneath the water surface in the designated device. To avoid that a leakage of the membrane leads to a wrong test result, an integrity test of the membrane bundle and the test system has to be carried out in advance. The integrity test is performed by pressing air through the membrane bundle that is closed on one side of the bundle. Air bubbles indicate a leakage of the membrane bundle or the test device. It has to be checked if the leakage can be associated with the wrong implementation of the membrane bundle in the test device or if a real membrane leakage is present. The membrane bundle has to be discarded if a leakage of the membrane is detected. The applied pressure of the integrity test has to be at least the same value as the applied pressure during the determination of the hydraulic permeability in order to ensure, that no leakage can occur during the measurement of the hydraulic permeability because of a too high applied pressure.

Sieving coefficient of IgG in aqueous solution

The sieving coefficient of IgG is determined by dead-end filtration of a solution of 5 g/l IgG in PBS buffer.

• The module is first washed with distilled water (10 ml at a flow rate of 0.04 to 1.5 ml/min).

• Then 100 ml of the IgG solution are filtered through the module at a flow rate of 0.7 ml/min.

• A sample of the ultrafiltrate is collected every 15 minutes and analyzed for IgG content using the UF-EW method.

• Overall filtration time is 2.5 hours.

Gold particle retention (GPR)

The module is first flushed with 6 ml of an alkaline wash solution (0.7 g Triton® X100 in 0.3 M NaOH) and subsequently with 10 ml of water at a flow rate of 0.2 ml/min.

A solution comprising gold particles having a diameter of 18-21 nm (AGP-HA 20, Asahi Kasei) is diluted with SDS (sodium dodecylsulfate solution) in a proportion of 1:9. This solution is pumped through the mini-module at a flow rate of 0.25 ml/min.

The particle content in the feed, the filtrate, and the retentate is determined photometrically by measuring extinction at 526 nm, e.g. using a Ultrospec™ 3000 pro photometer.

The GPR value is calculated according to equation (4)

$$GPR = \log_{10}\left(\frac{A}{F}\right) \qquad\qquad (4)$$

with

A being the extinction of the feed solution;
F being the extinction of the filtrate;

**Example 1**

Preparation of a solution of cellulose in an ionic liquid

A 7 wt.-% solution of cellulose in 1-ethyl-3-methylimidazolium acetate (EMIM Oac) was prepared by dissolving cotton linters (Linters V10, Milouban (M.C.P.) Ltd., M.P.O Ashrat 25201 Israel) in EMIM Oac using a LUK 0,75 kneader (Coperion Group GmbH, Stuttgart, Germany). The kneader was operated at 80 °C and 60 U/min for 21 h. The resulting solution had a viscosity of 237,900 mPa·s. The solution was degassed under vacuum (100 mbar).

**Example 2**

Preparation of a flat sheet membrane

The solution prepared in Example 1 was heated to 80 °C and cast onto a glass plate. A film was prepared using a doctor blade with a 70 $\mu$m gap at a speed of 12 mm/s. The glass plate was immersed for 5 minutes in a mixture of 35 wt.% EMIM OAc and 65 wt.% water having a temperature of 23 °C to precipitate the film. The membrane obtained had a thickness of about 60 $\mu$m.

The membrane was subsequently immersed in a glycerol/water (50/50 vol.%) bath for 30 minutes and then dried at 60 °C under reduced pressure for 1 hour.

The membrane showed a Lp of $61.8 \cdot 10^{-4}$ $cm^3/(cm^2 \cdot bar \cdot sec)$, a sieving coefficient for IgG of 0.96, and a GPR of 2.4.

**Example 3**

Preparation of a hollow fiber membrane

A 6 wt.-% solution of cellulose in EMIM OAc having a viscosity of 608,300 mPa·s was heated to 65 °C and extruded through the outer ring slit of a spinneret with two concentric openings into a coagulation bath containing water. Water was used as the center fluid and extruded through the inner opening of the spinneret. The temperature of the spinneret was 60 °C, the temperature of the coagulation bath was 40 °C. The fibers were spun at a speed of 5 m/min. The fiber obtained had an inner diameter of 360 $\mu$m and a wall thickness of 42 $\mu$m.

Water was drained from the fibers by centrifugation, and the fibers were subsequently immersed in a glycerol/water (50/50 vol.%) bath for 1 hour and then dried at 60 °C under reduced pressure for 1 hour.

Mini-modules comprising 6 fibers (corresponding to a membrane surface of about 10 $cm^2$) were prepared as described above.

The module showed a Lp of $106 \cdot 10^{-4}$ $cm^3/(cm^2 \cdot bar \cdot sec)$, a sieving coefficient for IgG of 0.99, and a GPR of 2.6.

**Claims**

1.  A copper-free membrane comprising cellulose, having a hydraulic permeability of from $40 \cdot 10^{-4}$ to $500 \cdot 10^{-4}$ cm$^3$/(CM$^2$·bar·sec), a sieving coefficient for IgG of greater than 0.8 and a GPR of greater than 1.4.

2.  The membrane of claim 1 in the form of a hollow fiber.

3.  The membrane of claim 1 in the form of a flat sheet.

4.  The membrane of any one of claims 1 to 3, prepared from a solution of cellulose in an ionic liquid.

5.  A process for preparing the membrane of claim 2, comprising the steps of

    (a) extruding a solution of cellulose in an ionic liquid through an outer ring slit of a nozzle with two concentric openings;
    (b) extruding a liquid or gaseous fluid which is chemically inert with respect to cellulose through the inner opening of the nozzle;
    (c) washing the membrane obtained in step (b);
    (d) treating said membrane with a pore stabilizer; and
    (e) optionally, drying said membrane and, optionally, sterilizing said membrane.

6.  A process for preparing the membrane of claim 3, comprising the steps of

    (a) extruding a solution of cellulose in an ionic liquid in order to form a flat sheet membrane;
    (b) solidifying the membrane obtained in step (a) by a phase inversion process by contacting the extruded solution with a liquid or gaseous fluid which is chemically inert with respect to cellulose;
    (c) washing the membrane obtained in step (b);
    (d) treating the membrane with a pore stabilizer; and
    (e) optionally, drying said membrane and, optionally, sterilizing said membrane.

7.  The process of claims 5 or 6, wherein the pore stabilizer is glycerol or a mixture of water and glycerol.

8.  The process of claims 5 or 6, wherein the ionic liquid comprises cations selected from the group consisting of methyl-tri-n-butylammonium, methylimidazolium, 1-ethyl-3-methylimidazolium, 1-butyl-3-methylimidazolium, 1-ethyl-2,3-dimethylimidazolium, 1,2,3-trimethylimidazolium, and 1,2,4-trimethylpyrazolium, and anions selected from the group consisting of acetate, ethylsulfate, methylsulfate, thiocyanate, hydrogensulfate, tetrachloroaluminate, and chloride.

9.  The process of claim 8, wherein the ionic liquid comprises 1-ethyl-3-methylimidazolium acetate.

10. A device for removing virus particles from a liquid, comprising a membrane according to any one of claims 1 to 4.

11. A method of removing virus particles from a liquid, comprising filtration of the liquid through a membrane according to any one of claims 1 to 4.

12. The method of claim 11, wherein the filtration is normal-flow filtration (NFF).

13. The method of claim 11, wherein the filtration is tangential-flow filtration (TFF).

14. Use of a membrane according to any one of claims 1 to 4 in a virus filter.

Europäisches
Patentamt

European
Patent Office

Office européen
des brevets

**EUROPEAN SEARCH REPORT**

Application Number

EP 08 02 2114

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2004/232062 A1 (BOIVIN DIDIER [FR] ET AL) 25 November 2004 (2004-11-25) <br> * the whole document * | 1-14 | INV. <br> C08J5/18 <br> C08L1/02 <br> B01D63/02 |
| A | G. CLOUGH AND C.C. MICHEL: "Quantitative coparisons of hydraulic permeability and edothelial intercellular cleft dimensions in single frog cappilaries" JOURNAL OF PHYSIOLOGY, vol. 405, 1988, pages 563-576, XP002526766 <br> * the whole document * | 1-14 | |
| A | M. RAFF ET AL.: "Advanced modeling of highflux hemodialysis" JOURNAL OF MEMBRANE SCIENCE, vol. 216, 2003, pages 1-11, XP002526767 <br> * the whole document * | 1-14 | |
| A | T. HONGO-HIRASAKI ET AL.: "Removal of small viruses (parovirus) from IgG solution by virus removal filter Plavonal 20N" JOURNAL OF MEMBRANE SCIENCE, vol. 278, 5 December 2005 (2005-12-05), pages 3-9, XP002526768 <br> * the whole document * | 1-14 | |
| A | J.F.STEVENSON ET AL.: "Hydraulic Permeability of Hollow-Fiber Membranes" JOURNAL OF BIOMEDICAL MATERIALS RESEARCH, vol. 12, 1978, pages 401-419, XP002526769 <br> * the whole document * | 1-14 | TECHNICAL FIELDS SEARCHED (IPC) <br><br> C08J <br> C08L <br> B01D |
| X | CN 101 234 297 A (UNIV TIANJIN POLYTECHNIC [CN]) 6 August 2008 (2008-08-06) <br> * abstract * <br><br> -/-- | 1-14 | |

The present search report has been drawn up for all claims

| Place of search | Date of completion of the search | Examiner |
|---|---|---|
| Munich | 7 May 2009 | olde Scheper, Bernd |

EPO FORM 1503 03.82 (P04C01)

Europäisches
Patentamt

European
Patent Office

Office européen
des brevets

**EUROPEAN SEARCH REPORT**

Application Number

EP 08 02 2114

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 101 240 454 A (FUJIAN HONGYUAN GROUP CO LTD [CN]) 13 August 2008 (2008-08-13) * abstract * ----- | 1-14 | |
| X | CN 101 284 913 A (XIAOSHAN GAO [CN]) 15 October 2008 (2008-10-15) * abstract * ----- | 1-14 | |

TECHNICAL FIELDS
SEARCHED      (IPC)

The present search report has been drawn up for all claims

| Place of search | Date of completion of the search | Examiner |
|---|---|---|
| Munich | 7 May 2009 | olde Scheper, Bernd |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 08 02 2114

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2009

| Patent document cited in search report | | Publication date | Patent family member(s) | Publication date |
|---|---|---|---|---|
| US 2004232062 | A1 | 25-11-2004 | NONE | |
| CN 101234297 | A | 06-08-2008 | NONE | |
| CN 101240454 | A | 13-08-2008 | NONE | |
| CN 101284913 | A | 15-10-2008 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0798003 A2
- JP 8187086 A
- EP 0302949 A1
- WO 2003029329 A
- DE 102005062608 A1

- US 20080164440 A1
- CN 1491974 A
- CN 101089249 A
- CN 101089250 A

**Non-patent literature cited in the description**

- *J. Membr. Sci.,* 2006, vol. 278, 3